# EUROPÄISCHE PATENTANMELDUNG

(11) **EP 2 548 609 A1**
(43) Veröffentlichungstag der Anmeldung: **23.01.2013**
(21) Anmeldenummer: 12174548.3
(22) Anmeldetag: 02.07.2012
(51) Int. Cl.: A61N 1/05

(54) **Unipolare Mehrzweck-Elektrodenleitung und Stimulations- und Defibrillationsanordnung**

(30) Priorität: 21.07.2011 US 201161510083 P
(71) Anmelder: BIOTRONIK SE & Co. KG, 12359 Berlin (DE)
(72) Erfinder: Dörr, Thomas, 12437 Berlin (DE)
(74) Vertreter: Lindner-Vogt, Karin L.

(57) **Zusammenfassung**

Unipolare Mehrzweck-Elektrodenleitung, mit einem Leitungskörper, einem unipolaren Stecker, einer am Leitungskörper angebrachten Defibrillationselektrode und Stimulations- und Abfühlelektrode, die über eine gemeinsame Elektrodenzuleitung mit dem unipolaren Stecker verbunden sind, wobei die Defibrillationselektrode derart durch mindestens ein spannungsabhängiges Bauelement derart mit der Elektrodenzuleitung verbunden ist, dass die Verbindung nur im Ansprechen auf das Anlegen einer Defibrillationsspannung am Stecker niederohmig ist.

## Beschreibung

Die Erfindung betrifft eine unipolare Mehrzweck-Elektrodenleitung, mit einem Leitungskörper, einem unipolaren Stecker, einer am Leitungskörper angebrachten Defibrillationselektrode und Stimulations- und Abfühlelektrode.

Implantierbare Defibrillatoren oder Kardioverter (ICD) sind seit langem bekannt und im klinischen Einsatz und seit Jahrzehnten Gegenstand permanenter technischer Weiterentwicklung. In noch stärkerem Maße gilt dies für implantierbare Herzschrittmacher und die zugehörigen Elektrodenleitungen. Als spezielle Geräteklasse haben sich auch kombinierte Herzstimulations- und Defibrillationsanordnungen, einschließlich der speziell hierfür entwickelten Elektrodenleitungen (ICD-Elektroden), auf dem Gerätenmarkt und in der klinischen Praxis etabliert. Derartige Kombinationsgeräte werden als Herzschrittmacher zur kardialen Resynchronisations-Therapie mit Defibrillator, abgekürzt als CRT-D-Geräte, bezeichnet.

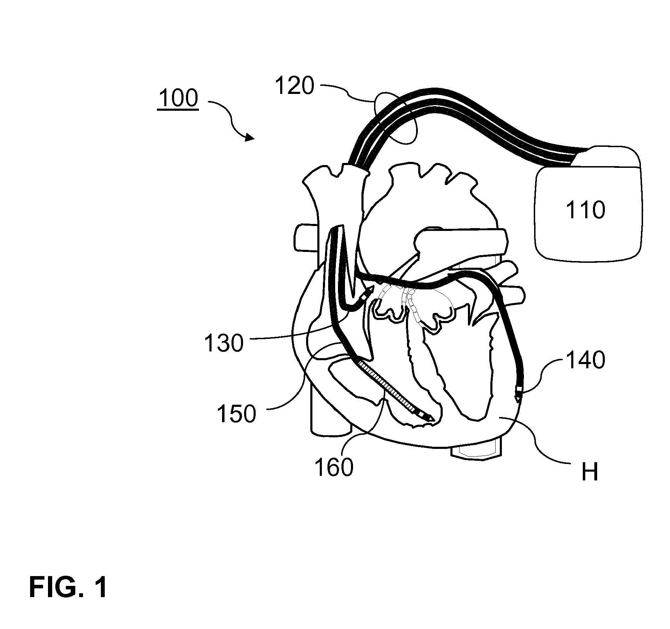

Fig. 1 zeigt eine schematische Darstellung einer solchen Anordnung 100 mit in das Herz H eines Patienten verlegten Elektroden. Demnach ist ein Herzstimulations- und Defibrillationsgerät 110 über eine Elektrodenleitung 120 mit dem Herzen H verbunden, die drei Leitungszweige bzw. Elektrodenzuleitungen 130, 140 und 150 umfasst. Alle Leitungszweige tragen an oder nahe ihrem distalen Ende (nicht einzeln bezeichnete) Abfühl- bzw. Stimulationselektroden, und der Leitungszweig 150 trägt zudem eine langgestreckte Defibrillationselektrode 160. In der gezeigten Anordnung ist der Leitungszweig 130 im rechten Atrium und der Leitungszweig 140 im linken Ventrikel des Herzens H verlegt, und der die Defibrillationselektrode 160 tragende Leitungszweig 150 ist im rechten Ventrikel (RV) verlegt.

Die derzeit verfügbaren ICD-Elektrodenleitungen haben mehrere, voneinander isolierte Zuleitungsdrähte und mehrere oder multipolare Steckkontakte zwischen der Elektrodenleitung und dem Impulsgenerator. Dies hat zur Folge, dass diese Elektrodenleitungen teuer in der Herstellung und anfällig für Defekte sind. Weiterhin sind die Anschlussblöcke der Pulsgeneratoren groß und teuer, und die Anwender können u.U. die Elektroden fehlerhaft anschließen. Zusätzlich bedingen die erforderlichen Hochspannungsisolationsabstände innerhalb der Elektrodenleitung einen Mindestdurchmesser der Elektroden. Damit geht Bauraum für Abschirmmaßnahmen zur Herstellung MRT-kompatibler ICD-Elektroden verloren bzw. waren Optionen von Niederspannungselektroden nicht auf Hochspannungselektroden anwendbar.

Der Erfindung liegt die Aufgabe zugrunde, eine verbesserte Elektrodenleitung der eingangs charakterisierten Art bereit zu stellen, die insbesondere einfach aufgebaut und handhabbar und hochgradig praxistauglich ist

Die Aufgabe wird durch eine unipolare Mehrzweck-Elektrodenleitung mit den Merkmalen des Anspruchs 1 gelöst. Des Weiteren wird eine implantierbare Stimulations- und Defibrillationsanordnung bereitgestellt, die mit einer solchen neuartigen Elektrodenleitung gebildet ist. Zweckmäßige Fortbildungen des Erfindungsgedankens sind Gegenstand der abhängigen Ansprüche.

Die Erfindung schließt den Gedanken ein, die Defibrillationselektrode einer Elektrodenleitung der in Rede stehenden Art bei Nichtgebrauch effektiv von der gemeinsamen Zuleitung, die sie sich mit einer Stimulations- und Abfühlelektrode teilt, elektrisch zu trennen. Gleichzeitig ist zu gewährleisten, dass sie im Bedarfsfall, also bei Ausgabe eines Schockimpulses, gleichwohl wirksam werden kann, in diesem Fall also niederohmig an die Zuleitung angeschlossen ist. Schließlich gehört zur Erfindung der Gedanke, die Defibrillationselektrode derart durch mindestens ein spannungsabhängiges Bauelement ("Schaltelement") mit der Elektrodenzuleitung zu verbinden, dass die Verbindung nur im Ansprechen auf das Anlegen einer Defibrillationsspannung am Stecker niederohmig ist.

Es sei hier darauf hingewiesen, dass es sich bei der vorgeschlagenen Leitung um eine unverzweigte bzw. einsträngige Elektrodenleitung, aber auch um eine verzweigte Leitung handeln kann, wobei im letzterem Falle die erfindungsgemäßen Merkmale mindestens einen der Leitungszweige charakterisieren.

In einer Ausführung der Erfindung ist vorgesehen, dass das oder jedes spannungsabhängige Schaltelement in die gemeinsame Elektrodenzuleitung integriert ist. Alternativ hierzu oder auch in Kombination mit dieser Ausführung kann vorgesehen sein, dass bei einem CRT-D-System das oder mindestens ein spannungsabhängiges Bauelement in den unipolaren Steckeranschluss des Stimulations- und Defibrillationsgerätes integriert ist.

In einer weiteren Ausführung der Erfindung ist das spannungsabhängige Bauelement ein Varistor, insbesondere SiC- oder ZnO-Varistor.

Eine weitere Ausführungsform sieht vor, dass dem spannungsabhängigen Bau- bzw. Schaltelement ein Widerstandselement zur Ausbildung eines Spannungsteilers derart zugeordnet ist, dass bei Anlegen eines Defibrillationsimpulses nur ein sehr kleiner Anteil der Energie über die Stimulations- und Abfühlelektrode abgegeben wird. Bevorzugt ist eine Ausgestaltung, bei der weniger als 5 % der Energie der Stimulations- und Abfühlelektrode zugeführt werden, und weiter bevorzugt ist ein noch kleinerer Anteil von weniger als 1 %.

In einer weiteren Ausführung der Erfindung ist die Defibrillationselektrode in Längsrichtung elektrisch segmentiert und jedem ihrer Segmente ein spannungsabhängiges Schaltelement zugeordnet. Hierbei sind die den Segmenten zugeordneten spannungsabhängigen Schaltelemente derart dimensioniert, dass sich bei Ausgabe eines Defibrillationsimpulses über die Defibrillationselektrode ein vorbestimmter Spannungsverlauf längs der Defibrillationselektrode einstellt. Es kann somit in Abhängigkeit von patientenspezifischen Konstellationen und der nach vollzogener Implantation konkret vorliegenden räumlichen Zuordnung zwischen Elektrodenleitung und reizbarem Herzgewebe eine zielgerichtet mehr oder weniger konstante Spannung über die Längserstreckung der Defibrillationselektrode und eine entsprechend variierende Energieabgabe im Einsatzfall eingestellt werden.

In einer weiteren Ausführung der Erfindung ist zwischen zwei Segmenten der Defibrillationselektrode, gegenüber beiden Segmenten isoliert, eine zusätzliche Stimulationselektrode eingefügt und dieser ein zusätzliches spannungsabhängiges Schaltelement zugeordnet. Diese Ausführung ermöglicht bspw. die bedarfsgerechte Platzierung einer Stimulationselektrode zur ventrikulären Stimulation (speziell LV-Stimulation) zusammen mit einer Schockelektrode großer Gesamtlänge auf einer unverzweigten Leitung oder einem einzelnen Leitungszweig einer verzweigten Leitung.

Gemäß einer weiteren Ausführung der Erfindung weist der Leitungskörper mindestens über einen Teil seiner Länge ein Isolationsmaterial mit erhöhter Wärmeleitfähigkeit zur Ableitung von Wärmeentwicklung an der Defibrillations- und/oder der Stimulations- und Abfühlelektrode auf. Diese Ausführung ermöglicht eine räumlich besser verteilte Wärmeübertragung an umgebendes Gewebe im Falle einer Erwärmung einer Zuleitung infolge von in einem starken äußeren Feld entstehenden Induktionsströmen. Da Materialien mit erhöhter Wärmeleitfähigkeit zumeist auch verschlechterte elektrische Isolationseigenschaften haben, ist diese Ausführung speziell bei der hier vorgeschlagenen unipolaren Elektrodenleitung praktikabel, weil es hier nicht auf eine hochwirksame elektrische Isolation zwischen einzelnen Elektrodenzuleitungen ankommt. In einer Ausgestaltung dieser Ausführung ist das Material mit erhöhter Wärmeleitfähigkeit im Wesentlichen über die gesamte Längserstreckung des Leitungskörpers vorgesehen.

In einer konstruktiven Ausführung der vorgeschlagenen Elektrodenleitung ist das oder mindestens ein spannungsabhängiges Schaltelement an ein Ende der Defibrillationselektrode räumlich direkt angefügt. Dies kann der Leitungsaufbau vereinfachen. Es versteht sich, dass entsprechende Ausgestaltungen auch bei der weiter oben erwähnten segmentierten Defibrillationselektrode, jeweils am Ende eines Segments, möglich sind.

In einer weiteren Ausführung ist das oder ein spannungsabhängiges Schaltelement als Keramik-Press- oder Sinterkörper ausgebildet. Diese Ausführung ist besonders vorteilhaft in Verbindung mit der vorgenannten, und zwar derart, dass ein zylindrischer oder hohlzylindrischer Keramik-Presskörper direkt an die Defibrillationselektrode angefügt oder - im Falle einer segmentierten Elektrode - jeweils ein Presskörper zwischen einzelnen Elektrodenabschnitten eingefügt ist.

Gemäß einer weiteren Ausführung der Erfindung weist die Defibrillationselektrode eine proximale Verlängerung auf, die mit einem Isolationsmantel des Leitungskörpers überdeckt ist. Hierbei hat die Verlängerung keinen elektrischen Kontakt mit anliegendem Gewebe, schirmt aber einen in ihrem Inneren verlaufenden Abschnitt der Elektrodenzuleitung ab. Durch diese Abschirmung wird der in starken äußeren Feldern auftretende Effekt der Entwicklung von Induktionsströmen in der Zuleitung und das Auftreten von für den Patienten potentiell gefährlichen Temperaturspitzen an Elektroden mit kleiner Oberfläche (also den Abfühl- bzw. Stimulationselektroden) reduziert.

Eine weitere Ausführung der vorgeschlagenen Elektrodenleitung ist mit mindestens einer weiteren Abfühl- und/oder Stimulationselektrode versehen, die über eine separate Elektrodenzuleitung angeschlossen ist. Wie weiter oben angemerkt, kann hierzu ein separater Leitungszweig vorgesehen sein, die zusätzliche(n) Elektrode(n) kann/können aber auch - neben den gemeinsam angeschlossenen Defibrillations- und Abfühl/Stimulationselektroden - auf einer einsträngigen Elektrodenleitung platziert sein. Geräteseitig ist dann vorgesehen, dass die Abfühl- und Stimulationskomponente des Stimulations- und Defibrillationsgerätes mindestens einen weiteren Ausgang aufweist, der mit einem weiteren Steckeranschluss verbunden ist.

Vorteile und Zweckmäßigkeiten der Erfindung ergeben sich im Übrigen aus der nachfolgenden Beschreibung von Ausführungsbeispielen anhand der Figuren. Von diesen zeigen:
- Fig. 1: eine schematische Darstellung einer Herzstimulations- und Defibrillationsanordnung,
- Fig. 2: eine schematische Darstellung einer erfindungsgemäßen Herzstimulations- und Defibrillationsanordnung,
- Fig. 3: eine schematische Darstellung zur Erläuterung einer Ausführungsform der Erfindung,
- Fig. 4: Beispiele von Varistorkennlinien,
- Fig. 5: eine schematische Darstellung zur Erläuterung einer weiteren Ausführungsform der Erfindung,
- Fig. 6: eine schematische Darstellung zur Erläuterung einer weiteren Ausführungsform der Erfindung,
- Fig. 7: eine schematische Darstellung zur Erläuterung einer weiteren Ausführungsform der Erfindung,
- Fig. 8: eine schematische Längsschnittdarstellung einer Elektrodenleitung gemäß einer weiteren Ausführungsform der Erfindung und
- Fig. 9: eine schematische Längsschnittdarstellung einer Elektrodenleitung gemäß einer weiteren Ausführungsform der Erfindung.

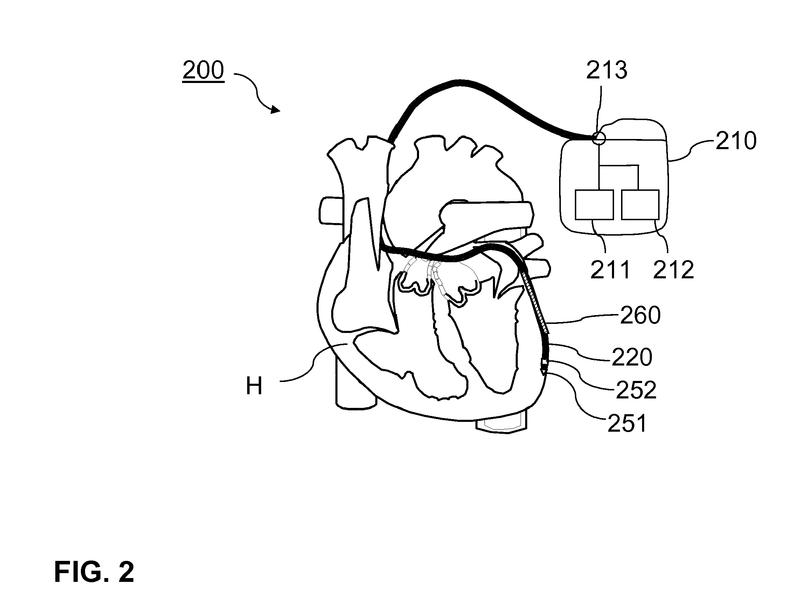

Fig. 2 zeigt eine mit einem Herzen H verbundene Herzstimulations- und Defibrillationsanordnung 200, die aus einem Herzstimulations- und Defibrillationsgerät (CRT-D-Gerät) 210 und einer einsträngigen ICD-Elektrodenleitung 220 gebildet ist. Die Elektrodenleitung 220 ist als unipolare Leitung ausgeführt und trägt an bzw. nahe ihrem distalen Ende eine Stimulationselektrode 251 und eine Abfühlelektrode 252 sowie proximal hiervon eine langgestreckte Defibrillationselektrode 260. Das CRT-D-Gerät 210 enthält eine Abfühl-und Stimulationskomponente 211 und eine Defibrillationskomponente 212, die beide unipolar mit einem Steckeranschluss 213 für die Leitung 220 verbunden sind.

Diese Anordnung geht von den Voraussetzungen aus, dass eine nur linksventrikuläre Stimulation mit entsprechend angepasster AV-Überleitungszeit der biventrikulären Stimulation nicht unterlegen ist und dass eine atriale Wahrnehmung z.B. über das Fernfeld-EKG, aufgezeichnet zwischen Schockwendel und Stimulatorgehäuse zuverlässig möglich ist. Ebenso kann das gezeigte System als konventionelles rechtsventrikuläres ICD-System eingesetzt werden.

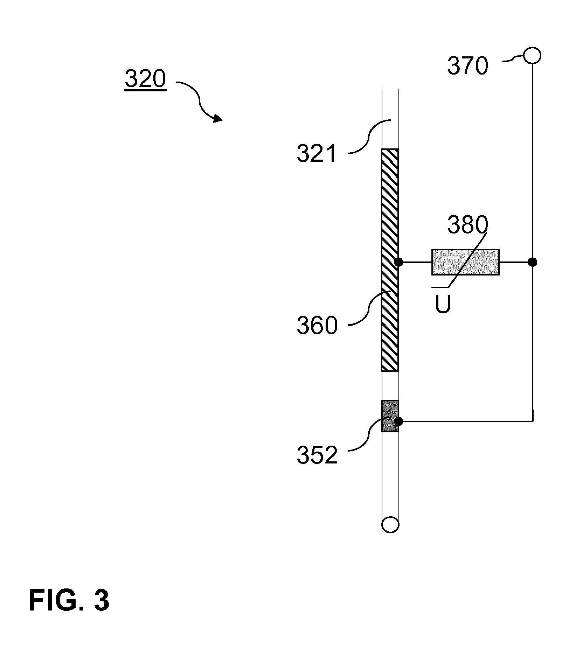

Fig. 3 zeigt schematisch den Endabschnitt (distalen Abschnitt) einer erfindungsgemäßen unipolaren Mehrzweck-Elektrodenleitung 320, die nahe ihrem distalen Ende eine Abfühl-und Stimulationselektrode 352 und proximal hiervon eine Defibrillationselektrode 360 auf einem Leitungskörper 321 trägt. Die Abfühl- und Stimulationselektrode 352 und Defibrillationselektrode 360 teilen sich eine Elektrodenzuleitung 370, und zwar derart, dass die Abfühl- und Stimulationselektrode 352 direkt und die Defibrillationselektrode 360 über ein Varistor-Bauelement 380 als spannungsabhängiges Schaltelement mit der gemeinsamen Zuleitung verbunden ist. Konstruktiv kann der Varistor 380 als integraler Bestandteil der Defibrillationselektrode 360 ausgeführt bzw. direkt an diese angefügt sein, oder er bildet an anderer Stelle einen Teil der Elektrodenzuleitung 370. Über den spannungsabhängigen Widerstand 380 wird eine Isolation der Schockwendel 360 für die Niederspannungsanwendung (Wahrnehmung/Stimulation) hergestellt und eine niederohmige Verbindung für die Schockabgabe realisiert.

Zur Reduktion einer MRT-induzierten Elektrodenerwärmung ist die Isolation des Leitungskörpers 321 im Bereich proximal der Schockwendel 360 so ausgeführt, dass gegenüber einer herkömmlichen Elektrodenleitung die Leitfähigkeit der Isolation erhöht ist. Dies ist möglich, da die Elektrode nur einen einzigen elektrischen Leiter im Inneren besitzt, der nicht gegenüber hochspannungsführenden Leitungen isoliert werden muss.

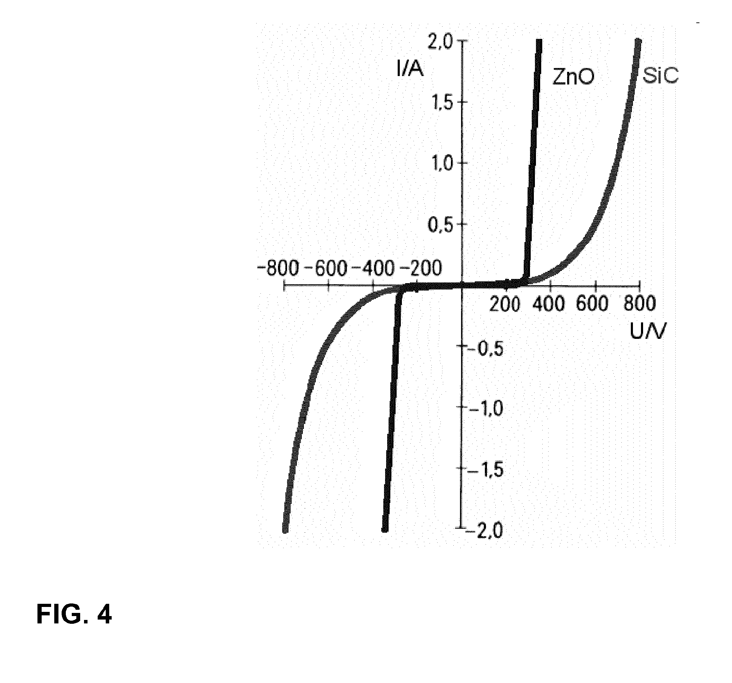

In Fig. 4 sind geeignete Kennlinien von typischen Varistoren dargestellt. Da Varistoren nicht abhängig von der Polarität sind, ist eine biphasische Schockabgabe möglich. Außerdem sind die Spannungsbereiche sehr gut für den Defibrillationsspannungsbereich einstellbar. Die Isolationseigenschaften bei kleinen Spannungen (Stimulationsspannungen bis 10V) sind ebenfalls ideal für die Anwendung in einem Elektrodensystem. Es versteht sich, dass hier nur ein Beispiel dargestellt ist und andere handelsübliche Varistoren mit anwendungsgerechten Parametern/Kennlinien ebenfalls bei der Ausführung der Erfindung eingesetzt werden können.

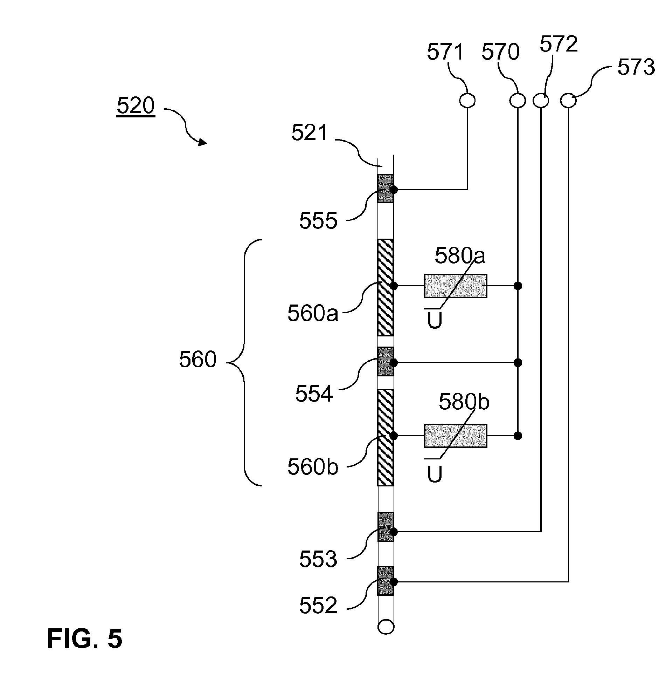

In Fig. 5 ist eine komplexere Realisierung einer mehrere Elektrodenzuleitungen umfassenden einsträngigen Mehrzweck-Elektrodenleitung 520 als Teil eines Single-Lead-CRT-D dargestellt. Hier verfügt die erfindungsgemäße Elektrodenleitung über ein 3 polares LV-Stimulationssystem 552, 553, 554, so dass eine Umschaltung der LV-Stimulationsvektoren möglich ist und so eine Anpassung von Stimulationsort- und Vektor an die patientenindividuellen Gegebenheiten ermöglicht wird. Für die Defibrillation kommen hier zwei Schockelektroden 560a, 560b, verbunden mit zwei Varistoren 580a, 580b mit einer der ventrikulären Zuleitungen zum Einsatz, so dass eine geometrische Positionierung einer LV-Stimulationselektrode 554 unterhalb der segmentierten Schockwendel 560 möglich ist. Weiterhin ist ein Ring 555 für die atriale Stimulation und Wahrnehmung vorgesehen. Bei den mit 570, 571, 572 und 573 bezeichneten Elementen handelt es sich um die Elektrodenzuleitungen zu den entsprechenden Elektroden.

Derartige LV-Elektroden mit 4 Zuleitungen können bereits realisiert werden und könnten mit einem einzigen IS-4/DF-4 Anschluss mit dem (nicht gezeigten) Herzstimulations- und Defibrillationsgerät verbunden werden.

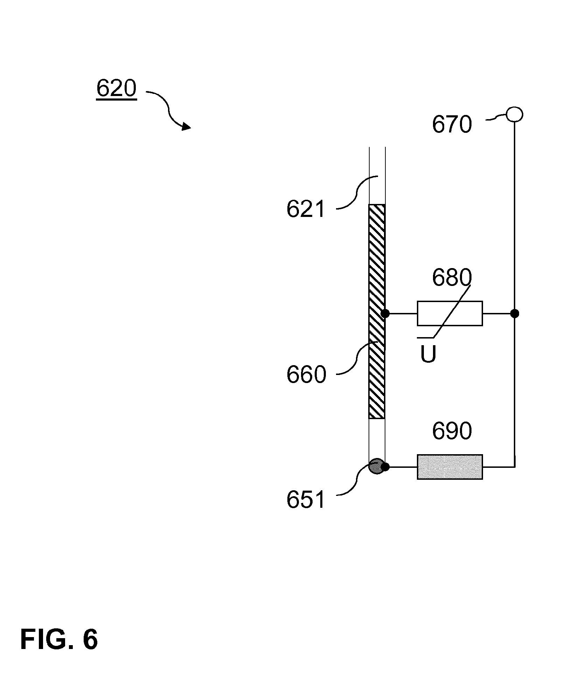

In Fig. 6 ist ein erweiterter Aufbau einer mit einem ICD/CRT-System verbundenen unipolaren Wahrnehmungs-/Stimulations-/Schockelektrode dargestellt. Die Elektrodenleitung 620 umfasst einen distalen Pol 651 zur Wahrnehmung und Stimulation im rechten oder linken Ventrikel, eine Schockwendel 660 zur Defibrillationsschockabgabe und eine einzige elektrische Zuleitung 670, wobei diese elektrische Zuleitung mit der Schockelektrode 660 über ein spannungsabhängiges Bauelement (z.B einen Varistor oder eine Supressordiode) 680 und mit dem Stimulationselektrodenpol 651 über einen (konstanten) Widerstand 690 verbunden ist.

Der spannungsabhängige Widerstand 680 und der konstante Widerstand 690 bilden dabei einen variablen Spannungsteiler. Solange die Therapiespannung unter der Schwellspannung des spannungsabhängigen Widerstandes liegt, wird die Therapieenergie nahezu vollständig an den Stimulations- und Wahrnehmungselektrodenpol 651 abgegeben. Dies ist für die Stimulation der Fall.

Überschreitet die Therapiespannung bei der Defibrillation deutlich die Schwellspannung des spannungsabhängigen Widerstandes 680, dann ergibt sich ein Spannungsteilerverhältnis, das den überwiegenden Anteil der Therapieenergie an die Schockwendel 660 ableitet. Dadurch wird erreicht, dass an dem kleinflächigem Stimulations- und Wahrnehmungspol 651 nur ein sehr geringer Energieumastz stattfinden kann und damit die Defibrillationstherapie wenig verlustbehaftet an der Schockwendel (630) umgesetzt und eine Gewebeschädigung am Stimulationselektrodenpol vermieden wird.

Das Teilerverhältnis sollte z.B. so eingestellt werden, dass mehr als 99% der Defibrillationsenergie über die Schockwendel abgegeben wird und gleichzeitig die Stimulationsfunktion nur geringfügig beeinträchtigt wird. Bei einer Dimensionierung des konstantem Widerstandes mit 500 Ohm und einem "On-Widerstand" <1 Ohm des spannungsabhängigem Widerstandes bei der Defibrillation und typischen Impedanzen für die Elektrodenpole von 500 Ohm für die Stimulationselektrode 651 und 50 Ohm für die Schockelektrode 660 ergibt sich ein Verhältnis der Energieabgabe von 99,5% via Schockelektrode und 0,5% via Stimulationselektrode.

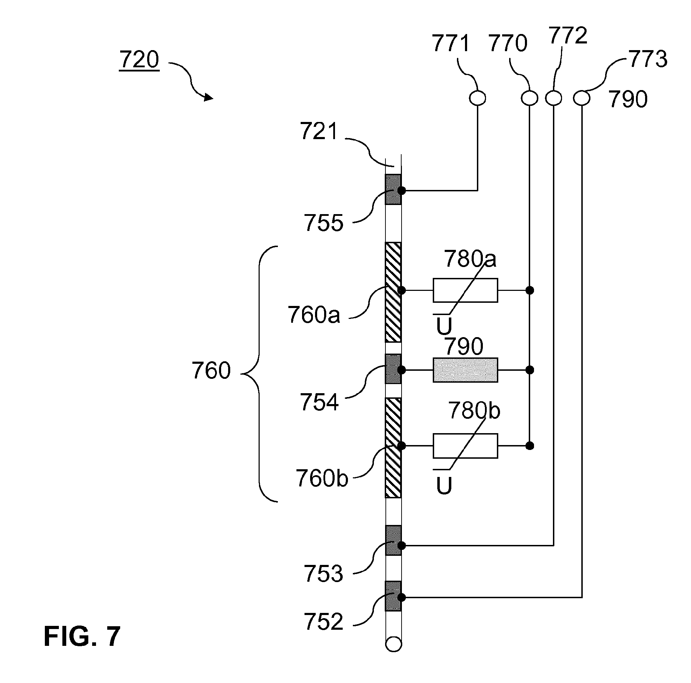

Fig. 7 zeigt eine Abwandlung der in Fig. 5 dargestellten und weiter oben beschriebenen Leitung mit mehreren Elektrodenzuleitungen unter Einbindung eines Spannungsteilers, wie vorstehend unter Bezugname auf Fig. 6 beschrieben. Die Bezugsziffern zur Bezeichnung der Teile dieser Elektrodenleitung 720 sind in Anlehnung an Fig. 5 vergeben, und die entsprechenden gleichen bzw. funktionsgleichen Teile werden hier nicht nochmals beschrieben, wobei die Nummern im Bereich 700 anstelle von 500 liegen. Hinzu gekommen ist der konstante Widerstand 790, über den die zwischen den Segmenten 760a, 760b der Schockelektrode 760 platzierte LV-Stimulationselektrode 754 mit der gemeinsamen (unipolaren) Elektrodenzuleitung 770 verbunden ist. Funktionen und Wirkungen des hiermit gebildeten Spannungsteilers entsprechen denen bei der vorstehend beschriebenen Ausführung nach Fig. 6.

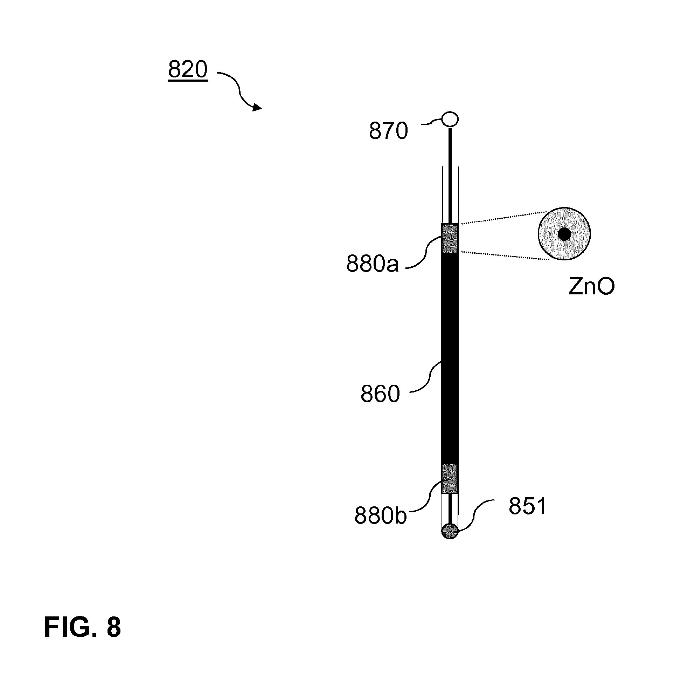

In Fig. 8 ist eine mögliche konstruktive Realisierung eine Elektrodenleitung gemäß Fig. 6 dargestellt. Zusätzlich ist hier zur Optimierung des elektrischen Feldes für die Defibrillation die Schockwendel 860 mit zwei spannungsabhängigen Widerständen 880a, 880b distal und proximal an die eine elektrische Zuleitung 870 angeschlossen. Die spannungsabhängigen Widerstände sind hier bevorzugt als Zink-Oxid-Sinterkörper ausgeführt, die im inneren Lumen mit der Zuleitung kontaktiert sind. Zur Realisierung des konstanten Widerstandes aus Fig. 6 (hier nicht dargestellt) wird die Tip-Elektrode 851 z.B. mit einen Konstantanmaterial angebunden.

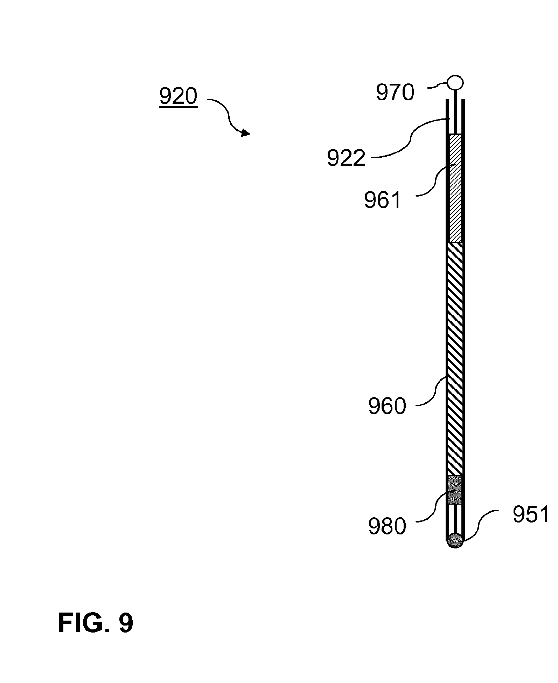

In Fig. 9 ist eine weitere mögliche Realisierung einer Elektrodenleitung gemäß Fig. 6 dargestellt. Die Schockwendel 960 ist auch hier mit einem spannungsabhängigen Widerstand 980 an die gemeinsame elektrische Zuleitung 970 angeschlossen, die gleichzeitig die Verbindung zur Tipelektrode 951 darstellt.

Zur besonders wirkungsvollen Abschirmung von MRT-Feldern ist hier die Schockwendel als Abschirmung im proximalen Bereich oberhalb der elektrisch notwendigen Defibrillationselektrode unterhalb der Isolation 922 mit einer Verlängerung 961 verlängert. So entsteht eine sehr wirkungsvolle und einfache MRT-Abschirmung, die in dem freigewordenem Bauraum der Elektrodenleitung gut untergebracht werden kann - insbesondere auch deshalb, weil keine Hochspannungsisolationsanforderungen bestehen. Die Isolierung 922 dieser Elektrodenleitung 920 kann im Übrigen - übe die gesamte Länge oder auch nur in dem Bereich, in dem sich die Verlängerung 961 befindet - aus einem Material mit erhöhter Wärmeleitfähigkeit gebildet sein, um erforderlichenfalls eine besser verteilte Abgabe von in den leitfähigen Teilen der Elektrode durch Wechselwirkung mit einem äußeren Wechselfeld entwickelter Wärme zu gewährleisten.

Die Ausführung der Erfindung ist nicht auf die oben beschriebenen Beispiele und hervorgehobenen Aspekte beschränkt, sondern ebenso in einer Vielzahl von Abwandlungen möglich, die im Rahmen fachgemäßen Handelns liegen.

## Patentansprüche

1. Unipolare Mehrzweck-Elektrodenleitung, mit einem Leitungskörper, einem unipolaren Stecker, einer am Leitungskörper angebrachten Defibrillationselektrode und Stimulations- und Abfühlelektrode, die über eine gemeinsame Elektrodenzuleitung mit dem unipolaren Stecker verbunden sind, wobei die Defibrillationselektrode derart durch mindestens ein spannungsabhängiges Bauelement derart mit der Elektrodenzuleitung verbunden ist, dass die Verbindung nur im Ansprechen auf das Anlegen einer Defibrillationsspannung am Stecker niederohmig ist.

2. Mehrzweck-Elektrodenleitung nach Anspruch 1, wobei das oder jedes spannungsabhängige Bauelement in die gemeinsame Elektrodenzuleitung integriert ist.

3. Mehrzweck-Elektrodenleitung nach Anspruch 1 oder 2, wobei das spannungsabhängige Bauelement ein Varistor, insbesondere SiC- oder ZnO-Varistor, ist.

4. Mehrzweck-Elektrodenleitung nach einem der vorangehenden Ansprüche, wobei dem spannungsabhängigen Bauelement ein Widerstandselement zur Ausbildung eines Spannungsteilers derart zugeordnet ist, dass bei Anlegen eines Defibrillationsimpulses nur ein sehr kleiner Anteil, insbesondere weniger als 5 %, weiter bevorzugt weniger als 1 %, der Energie über die Stimulations- und Abfühlelektrode abgegeben wird.

5. Mehrzweck-Elektrodenleitung nach einem der vorangehenden Ansprüche, wobei die Defibrillationselektrode in Längsrichtung elektrisch segmentiert und jedem ihrer Segmente ein spannungsabhängiges Bauelement zugeordnet ist, wobei die den Segmenten zugeordneten spannungsabhängigen Bauelemente derart dimensioniert sind, dass sich bei Ausgabe eines Defibrillationsimpulses über die Defibrillationselektrode ein vorbestimmter Spannungsverlauf längs der Defibrillationselektrode einstellt.

6. Mehrzweck-Elektrodenleitung nach Anspruch 5, wobei zwischen zwei Segmenten der Defibrillationselektrode, gegenüber beiden Segmenten isoliert, eine zusätzliche Stimulationselektrode eingefügt und dieser ein zusätzliches spannungsabhängiges Bauelement zugeordnet ist.

7. Mehrzweck-Elektrodenleitung nach einem der vorangehenden Ansprüche, wobei der Leitungskörper mindestens über einen Teil seiner Länge ein Isolationsmaterial mit erhöhter Wärmeleitfähigkeit zur Ableitung von Wärmeentwicklung an der Defibrillations- und/oder der Stimulations- und Abfühlelektrode aufweist.

8. Mehrzweck- Elektrodenleitung nach Anspruch 7, wobei das Material mit erhöhter Wärmeleitfähigkeit im Wesentlichen über die gesamte Längserstreckung des Leitungskörpers vorgesehen ist.

9. Mehrzweck-Elektrodenleitung nach einem der vorangehenden Ansprüche, wobei das oder mindestens ein spannungsabhängiges Bauelement an ein Ende der Defibrillationselektrode räumlich direkt angefügt ist.

10. Mehrzweck-Elektrodenleitungen nach einem der vorangehenden Ansprüche, wobei das oder ein spannungsabhängiges Bauelement als Keramik-Press- oder Sinterkörper ausgebildet ist.

11. Mehrzweck-Elektrodenleitung nach einem der vorangehenden Ansprüche, wobei die Defibrillationselektrode eine proximale Verlängerung aufweist, die mit einem Isolationsmantel des Leitungskörpers überdeckt ist, derart, dass die Verlängerung keinen elektrischen Kontakt mit anliegendem Gewebe hat, aber einen in ihrem Inneren verlaufenden Abschnitt der Elektrodenzuleitung abschirmt.

12. Mehrzweck-Elektrodenleitung nach einem der vorangehenden Ansprüche, mit mindestens einer weiteren Abfühl- und/oder Stimulationselektrode, die über eine separate Elektrodenzuleitung angeschlossen ist.

13. Implantierbare Stimulations- und Defibrillationsanordnung, mit einem Stimulations-und Defibrillationsgerät, welches eine Abfühl- und Stimulationskomponente und eine Defibrillationskomponente aufweist, wobei der Ausgang der Defibrillationskomponente und mindestens ein Ausgang und ein Eingang der Abfühl- und Stimulationskomponente gemeinsam mit einem unipolaren Steckeranschluss verbunden sind, und einer Mehrzweck-Elektrodenleitung nach einem der vorangehenden Ansprüche.

14. Stimulations- und Defibrillationsanordnung nach Anspruch 13, wobei die Abfühl-und Stimulationskomponente des Stimulations- und Defibrillationsgerätes mindestens einen weiteren Ausgang aufweist, der mit einem weiteren Steckeranschluss verbunden ist.

15. Stimulations- und Defibrillationsanordnung nach Anspruch 13 oder 14, wobei das oder mindestens ein spannungsabhängiges Bauelement in den unipolaren Steckeranschluss des Stimulations- und Defibrillationsgerätes integriert ist.
